(19) Europäisches Patentamt
European Patent Office
Office européen des brevets

(11) Veröffentlichungsnummer : **0 071 790**
**B1**

(12) # EUROPÄISCHE PATENTSCHRIFT

(45) Veröffentlichungstag der Patentschrift : **24.04.85**

(51) Int. Cl.$^4$ : **C 07 C149/40**, C 07 C148/00

(21) Anmeldenummer : **82106332.8**

(22) Anmeldetag : **15.07.82**

(54) **Neue Fluormethylthiobenzoylfluoride und Verfahren zu ihrer Herstellung.**

(30) Priorität : **01.08.81 DE 3130436**

(43) Veröffentlichungstag der Anmeldung : **16.02.83 Patentblatt 83/07**

(45) Bekanntmachung des Hinweises auf die Patenterteilung : **24.04.85 Patentblatt 85/17**

(84) Benannte Vertragsstaaten : **BE CH DE FR GB LI**

(56) Entgegenhaltungen :
**EP-A- 0 039 157**
**DE-A- 2 117 650**
**HOUBEN WEYL "Methoden der organischen Chemie" 4. Auflage, Band V/3 "Halogenverbindungen" 1962, GEORG THIEME VERLAG, Stuttgart Seiten 119 bis 123**
**Chemical Abstracts Band 49, Nr. 12 25 Juni 1955 Columbus, Ohio, USA L.M. YAGUPOL'SKII et al. "Phenyl trifluoromethyl sulfides and phenyl trifluoromethyl sulfones with substituents in the p-position" Spalte 8172d**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Varwig, Juergen, Dr.**
**Am Goetzenberg 1**
**D-6900 Heidelberg (DE)**
Erfinder : **Hamprecht, Gerhard, Dr.**
**Rote-Turm-Strasse 28**
**D-6940 Weinheim (DE)**

**EP 0 071 790 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Fluormethylthiobenzoylfluoride und ein Verfahren zu ihrer Herstellung durch Umsetzung von Trichlormethylthiobenzoylchloriden mit Fluorwasserstoff bei 10 bis 130 °C.

Darstellungen von Trifluormethylphenylsulfiden aus den entsprechenden Trichlor-Verbindungen mit Hilfe von Antimontrifluorid oder Fluorwasserstoff sind bekannt (C.A. 49, (1955) Nr. 12, Spalte 8 172 g ; FR-PS 820 796).

Die Darstellung von Trifluormethylthio-substituierten Benzoylchloriden wird bisher auf umständlichem Wege über das aus dem Trifuormethylthionitrobenzol durch Reduktion hergestellte Anilinderivat, das weiter zum entsprechenden Benzonitril und der Benzoesäure umgesetzt und anschließend mit Thionylchlorid chloriert wird, durchgeführt (C.A. loc. cit.).

Weitere Methoden zu Trifluormethylthiobenzoesäurederivaten, die jedoch bis zur Stufe der Säurechloride noch weiteren Reaktionsschritten unterworfen werden müssen, beinhalten die Umsetzung von Jod- oder Brom-substituierten Nitrobenzolen oder Benzoesäureestern mit Quecksilber- oder Kupfertrifluormethylsulfid (US-PS 4 020 169 J. Org. Chem. *41,* 1 644 (1976)). Die für die Reaktionen notwendigen Quecksilberverbindungen sind teuer und giftig, sowie umständlich über das Trichlormethylthiosulfenylchlorid herzustellen. Das Kupfertrifluormethylsulfid wird aus der Quecksilberverbindung synthetisiert.

Es ist aus Houben-Weyl, Methoden der organischen Chemie, 4. Auflage (1962), Seite 123, bekannt, daß Trichlormethylphenyl-äther mit Flußsäure in die Difluor-chlor-methyl- und Trifluormethylverbindungen überführt werden können, z. B. durch Umsetzung bei 120-160 °C und unter einem Druck von 41-51 bar. Auch Thioäther, z. B. Trichlormethyl-phenyl-sulfid bei 50-100 °C, können mit Flußsäure fluoriert werden.

Das in der deutschen Offenlegungsschrift 21 17 650 beschriebene Verfahren liefert Fluormethoxybenzoylfluoride und zwar nur Mono- und Difluorverbindungen durch Umsetzung entsprechender Trichlormethoxibenzoylchloride bei 60 bis 130 °C unter Druck. Es wird angegeben, daß erst eine Umsetzung um etwa 130 °C einen gewissen Anteil an der Trifluorverbindung liefert. Beispiel 5 zeigt, daß bei 29-94 °C ein Gemisch von Mono- (11,8 %), Di- (79,6 %) und Trifluorverbindungen (5,6 %) entsteht.

Es wurde nun gefunden, daß man Fluormethylthiobenzoylfluoride der Formel

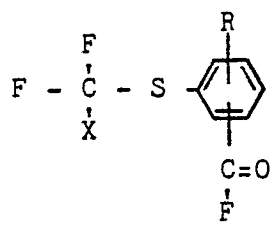

(I)

worin X ein Chloratom oder ein Fluoratom bedeutet, R ein Wasserstoffatom, ein Halogenatom oder einen aliphatischen Rest bezeichnet, vorteilhaft erhält, wenn man Trichlormethylthiobenzoylchloride der Formel

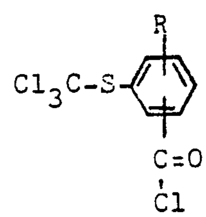

(II)

worin R die vorgenannte Bedeutung besitzt, mit 1,2 bis 100 Mol Fluorwasserstoff je Mol Ausgangsstoff II

a) zur Herstellung der Trifluormethylthiobenzoylfluoride mit einer Temperatur von 40 bis 130 °C und einem Druck von 2 bis 30 bar oder

b) zur Herstellung der Difluormonochlormethylthiobenzoylfluoride zwischen 10 und 40 °C und einem Druck von 1 bis 10 bar

umsetzt.

Weiterhin wurden die neuen Fluormethylthiobenzoylfluoride der Formel

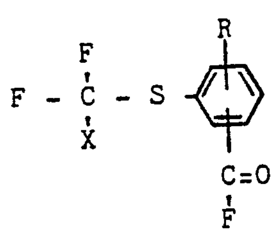

(I)

worin X ein Chloratom oder ein Fluoratom bedeutet, R ein Wasserstoffatom, ein Halogenatom oder einen aliphatischen Rest bezeichnet, gefunden.

Die Umsetzung kann im Falle der Verwendung von m-Trichlormethylthiobenzoylchlorid durch die folgenden Formeln wiedergegeben werden :

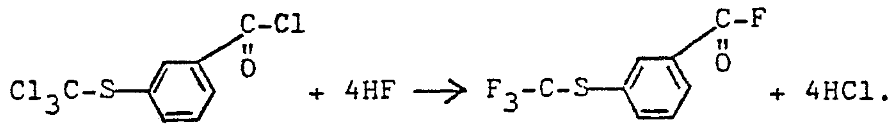

Im Hinblick auf den Stand der Technik liefert das Verfahren nach der Erfindung auf einfachem und wirtschaftlichem Wege die neuen Fluormethylthiobenzoylfluoride in guter Ausbeute und Reinheit. Alle diese vorteilhaften Eigenschaften sind mit Bezug auf den Stand der Technik überraschend.

Es ist überraschend, daß die beim erfindungsgemäßen Verfahren eingesetzten Ausgangsstoffe erheblich reaktiver sind und selektiver als die Sauerstoff-Verbindungen (DE-A-2 117 650) reagieren. So wurde nach Beispiel 1 und 2 (DE-A-2 117 650) bei einer Reaktionstemperatur von 90-100 °C aus 3-Trichlormethoxybenzoylchlorid nahezu ausschließlich 3-Difluorchlormethoxybenzoylfluorid erhalten, also nur teilweise Fluor eingeführt, währen nach dem erfindungsgemäßen Verfahren der Ausgangsstoff bereits bei tieferen Temperaturen (70 °C) das vollständig fluorierte 3-Trifluormethylthiobenzoylfluorid liefert (Beispiel 1). Der teilweise fluorierte Endstoff, z. B. das p-Chlordifluormethylthiobenzoylfluorid, wird erfindungsgemäß (Beispiel 3) in vergleichbarer Ausbeute von 82 % bereits bei 20 °C erhalten. Beispiel 3 von DE-A-2 117 650 im Verleich mit Beispiel 5 (erfindungsgemäß) zeigt erneut die unterschiedliche Reaktion.

Auch ist es überraschend, daß die Difluorchlormethoxyverbindungen (DE-A-2 117 650, Beispiele) nur unter Druck, hingegen die erfindungsgemäßen Stoffe auch drucklos (Beispiel 2 und 3) hergestellt werden können. Es war ebenfalls im Hinblick auf DE-A-2 117 650 überraschend, daß bei der erfindungsgemäßen Umsetzung die Trifluormethylverbindungen in hoher Ausbeute und nicht im Gemisch mit wesentlichen Mengen an Difluor- und Monofluormethylverbindungen erhalten werden. Das Verfahren nach DE-A-2 117 650 liefert entweder nur die Difluormethylverbindungen (Beispiele 1 bis 3) oder ein Gemisch der Difluor- und Monofluormethylverbindungen (Beispiel 4) oder ein Gemisch von Mono-, Di- und Trifluormethylverbindungen (Beispiel 5). Es liefert de Trifluormethylverbindung höchstens in einer Ausbeute von 5,6 % (Beispiel 5).

C.A. (loc. cit.) gibt zur Herstellung des Trifluormethylbenzoylchlorids nur ein Verfahren mit mehreren Stufen und unter Verwendung von Antimontrifluorid an. Aus Houben-Weyl (loc. cit.) konnte man nicht ersehen, in welcher Weise die sehr reaktive Chlorcarbonylgruppe die erfindungsgemäße Umsetzung beeinflußt. Entsprechend gibt Houben-Weyl (loc. cit.) keinen Hinweis darauf, ob und auf welche Weise alle 4 Chloratome der beim erfindungsgemäßen Verfahren eingesetzten Ausgangsstoffe durch Fluor ersetzt werden können. Daß die Reaktion (Houben-Weyl, loc. cit.) durch die Carboxylgruppe nicht gestört wird, gibt lediglich einen Hinweis darauf, daß zwar eine Trifluormethylverbindung entstehen kann, die Carboxylgruppe aber unverändert bleibt ; ein entsprechendes Verhalten der Chlorcarbonylgruppe mußte erwartet werden. Es war daher nicht zu vermuten, daß alle 4 Chloratome im Ausgangsstoff durch Fluoratome ersetzt werden können, und man hätte gerade im Hinblick auf Houben-Weyl (loc. cit.) nicht erwartet, daß Verbindungen mit nur 2 Fluoratomen an der Methylgruppe und einer Fluorcarbonylgruppe herstellbar sind.

Bevorzugte Ausgangsstoffe II und dementsprechend bevorzugte Endstoffe I sind solche, in deren Formeln X ein Chloratom oder Fluoratom bedeutet und R ein Wasserstoffatom, ein Bromatom, Chloratom oder einen Alkylrest mit 1 bis 4 Kohlenstoffatomen bezeichnet. Die vorgenannten Reste können noch durch unter den Reaktionsbedingungen inerte Reste, z. B. Alkylreste oder Alkoxireste mit 1 bis 4 Kohlenstoffatomen, substituiert sein.

So kommen z. B. als Ausgangsstoffe II in Frage : m-Trichlormethylthiobenzoylchloride und entsprechend durch Brom, Chlor, Methyl-, Ethyl-, Propyl-, Isopropyl-, Butyl-, Isobutyl-, sek.-Butyl-, tert.-Butylgruppen in m-, o- oder p-Stellung am Phenylkern substituierte Benzoylchloride ; entsprechende o- und p-Trichlormethylverbindungen.

Die Ausgangsverbindungen können miteinander mit stöchiometrischen Mengen oder zweckmäßig im Überschuß an Fluorwasserstoff, vorteilhaft im Verhältnis von 1,2 bis 100 Mol HF, vorzugsweise von 1,5 bis 20 Mol HF je Mol Trichlormethylthiobenzoylchlorid umgesetzt werden.

Die Umsetzung wird bei einer Temperatur von 10 bis 130 °C, für die Herstellung der Trifluormethyl-Verbindung von 40 bis 130 °C, insbesondere von 40 bis 80 °C, für die Herstellung der Difluormethyl-Verbindung zwischen 10 und 40 °C, insbesondere zwischen 10 und 30 °C, unter Druck oder drucklos, kontinuierlich oder diskontinuierlich durchgeführt.

Der Reaktionsdruck beträgt 1 bis 30 bar, für die Herstellung der Trifluormethyl-Verbindung 2 bis 30 bar, für die Herstellung der Difluormethyl-Verbindung 1 bis 10 bar. Das bei der Reaktion sich entwickelnde HCl-Gas wird dabei je nach gewünschtem Druck abgelassen.

Die Reaktion kann ohne oder mit Lösungsmitteln erfolgen. Als Lösungsmittel kommen z. B. in Frage : Halogenkohlenwasserstoffe, insbesondere Brom- und Chlorkohlenwasserstoffe, z. B. Tetrachloräthylen,

1,1,2,2- oder 1,1,1,2-Tetrachloräthan, Amylchlorid, Cyclohexylchlorid, 1,2-Dichlorpropan, Methylenchlorid, Dichlorbutan, Isopropylbromid, n-Propylbromid, Butylbromid, Chloroform, Bromoform, Äthyljodid, Propyljodid, Chlornaphthalin, Dichlornaphthalin, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid ; aliphatische oder cycloaliphatische Kohlenwasserstoffe, z. B. Pentan, Heptan, α-Pinen, Pinan, Nonan, Benzinfraktionen innerhalb eines Siedepunktintervalls von 70 bis 190 °C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan ; und entsprechende Gemische. Zweckmäßig verwendet man das Lösungsmittel in einer Menge von 80 bis 10 000 Gewichtsprozent, vorzugsweise von 100 bis 600 Gewichtsprozent, bezogen auf Ausgangsstoff II.

Die Umsetzung kann ohne oder mit Katalysatoren wie Antimon-, Eisen-, Kobalt-, Nickel-, Mangan-, Titan-, Aluminium-, Borsowie Chrom-Verbindungen, z. B. die Fluoride, durchgeführt werden.

Die Reaktion kann wie folgt durchgeführt werden : Fluorwasserstoff und das Trichlormethylthiobenzoylchlorid, gegebenenfalls im Gemisch mit Lösungsmitteln und/oder Katalysatoren werden bei der Reaktionstemperatur 0,5 bis 10 Stunden gerührt und die entstehende Salzsäure bei einem Druck zwischen 1 und 30 bar ausgekreist. Die überschüssige Flußsäure wird abdestilliert und kann im Kreis gefahren werden. Der Endstoff wird in üblicher Weise durch Destillation gereinigt. Es kann auch zweckmäßig sein, das Produkt mit Wasser mit oder ohne Zusatz eines mit Wasser nicht mischbaren Lösungsmittels zu waschen.

Die nach dem Verfahren der Erfindung erhältlichen Fluormethylthiobenzoylfluoride sind wertvolle Ausgangsstoffe für die Herstellung von Pflanzenschutzmitteln. So erhält man beispielsweise durch Umsetzung mit Anthranilsäuren Benzoxazinone mit ausgeprägter herbizider Wirkung (DE-OS 29 14 915).

Die in den folgenden Beispielen aufgeführten Teile bedeuten Gewichtsteile.

### Beispiel 1

In einem V4A-Autoklaven werden 1 540 Teile m-Trichlormethylthiobenzoylchlorid und 1 900 Teile Fluorwasserstoff 6 Stunden bei 70 °C gerührt. Durch Abgasen der entstehenden Salzsäure wird der Druck bei 10 bar gehalten. Die Apparatur wird entspannt, die überschüssige Flußsäure abdestilliert und das flüssige Reaktionsprodukt destilliert. Ausbeute 891 Teile (75 % der Theorie) m-Trifluormethylthiobenzoylfluorid, $n_D^{25}$ = 1,477 7 ; Kp (5 mbar) 60 °C.

### Beispiel 2

In einem Teflonkolben mit Rückflußkühler werden 650 Teile p-Trichlormethylthiobenzoylchlorid und 500 Teile Fluorwasserstoff 7 Stunden unter Rückfluß (20 °C) drucklos erhitzt. Die Aufarbeitung ergibt nach dem Abdestillieren der Flußsäure 441 Teile (83 % der Theorie) p-Chlordifluormethylthiobenzoylfluorid, Kp (16 mbar) 106-108 °C ; $n_D^{25}$ = 1,514 4.

$^{13}$C-NMR ($CDCl_3$) δ (ppm) (intern TMS) :

| | | |
|---|---|---|
| $C_1$ 127,22 (D) | $C_4$ 135,11 (S) | $C_7$ 156,61 (D) |
| $C_2$ 132,25 (S) | $C_5$ 136,04 (S) | $C_8$ 130,05 (T) |
| $C_3$ 136,04 (S) | $C_6$ 132,25 (S) | |

### Beispiel 3

In einem Teflonkolben mit Rückflußkühler werden 2 600 Teile m-Trichlormethylthiobenzoylchlorid und 2 000 Teile Fluorwasserstoff 7 Stunden bei 1 bar unter Rückfluß gerührt (20 °C). Nach dem Abdestillieren der Flußsäure erhält man 1 965 Teile (82 % der Theorie) m-Chlordifluormethylthiobenzoylfluorid, Kp (0,2 mbar) 47-50 °C, $n_D^{25}$ = 1,508 9.

### Beispiel 4

In einem Autoklaven werden 270 Teile 3-Trichlormethylthio-4-chlorbenzoylchlorid und 500 Teile wasserfreie Flußsäure 8 Stunden bei 70 °C mit 20 bar gerührt. Das Produktgemisch wird auf Eiswasser gegeben, mit Methylenchlorid extrahiert und mit Wasser gewaschen. Ausbeute : 130 Teile (62 % der Theorie) 3-Trifluormethylthio-4'-chlorbenzoylfluorid, Kp (0,1 mbar) 41 °C.

$^{13}$C-NMR ($CDCl_3$) (ppm) (intern TMS) :

| | | |
|---|---|---|
| $C_1$ 125,1 (D) | $C_4$ 147,9 (S) | $C_7$ 115,7 (D) |
| $C_2$ 140,8 (S) | $C_5$ 131,9 (S) | $C_8$ 129,2 (Q) |
| $C_3$ 126,5 (S) | $C_6$ 134,7 (S) | |

### Beispiel 5

Analog Beispiel 1 wird die Umsetzung, jedoch mit 1 540 Teilen p-Trichlormethylthiobenzoylchlorid

und 1 900 Teilen Flußsäure, durchgeführt. Man erhält p-Trifluormethylthiobenzoylfluorid vom Kp (16 mbar) 85-89 °C in einer Ausbeute von 70 % der Theorie ; $n_D^{25} = 1{,}482\,9$.

**Patentansprüche**

1. Verfahren zur Herstellung von Fluormethylthiobenzoylfluoriden der Formel

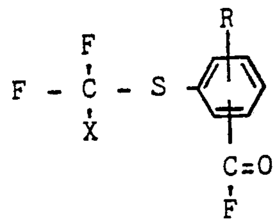

(I)

worin X ein Chloratom oder ein Fluoratom bedeutet, R ein Wasserstoffatom, ein Halogenatom oder einen aliphatischen Rest bezeichnet, dadurch gekennzeichnet, daß man Trichlormethylthiobenzoylchloride der Formel

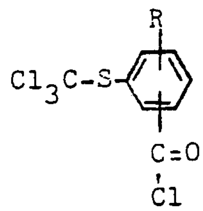

(II)

worin R die vorgenannte Bedeutung besitzt, mit 1,2 bis 100 Mol Fluorwasserstoff je Mol Ausgangsstoff II

a) zur Herstellung der Trifluormethylthiobenzoylfluoride mit einer Temperatur von 40 bis 130 °C und einem Druck von 2 bis 30 bar oder

b) zur Herstellung der Difluormonochlormethylthiobenzoylfluoride zwischen 10 und 40 °C und einem Druck von 1 bis 10 bar

umsetzt.

2. Fluormethylthiobenzoylfluoride der Formel

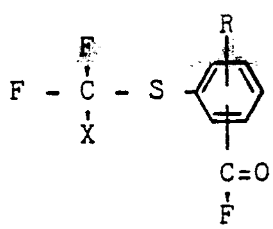

(I)

worin X ein Chloratom oder ein Fluoratom bedeutet, R ein Wasserstoffatom, ein Halogenatom oder einen aliphatischen Rest bezeichnet.

**Claims**

1. A process for the preparation of a fluoromethylthiobenzoyl fluoride of the formula

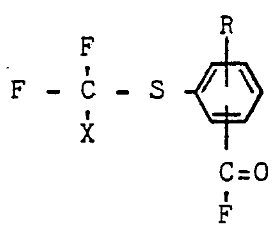

(I)

where X is chlorine or fluorine and R is hydrogen, halogen or an aliphatic radical, wherein one mole of a trichloromethylthiobenzoyl chloride of the formula

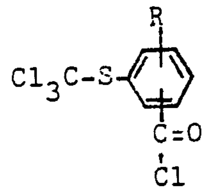

(II)

where R has the above meanings, is reacted with from 1.2 to 100 moles of hydrogen fluoride

a) at from 40 to 130 °C and under from 2 to 30 bar for the preparation of the trifluoromethylthiobenzoyl fluoride, or

b) at from 10 to 40 °C and under a pressure of from 1 to 10 bar for the preparation of the difluoromonochloromethylthiobenzoyl fluoride.

2. A fluoromethylthiobenzoyl fluoride of the formula

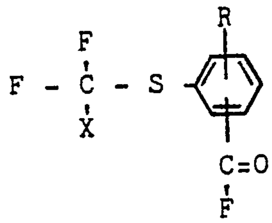

(I)

where X is chlorine or fluorine, and R is hydrogen, halogen or an aliphatic radical.

## Revendications

1. Procédé de préparation de fluorures de fluorométhylthiobenzoyle de formule

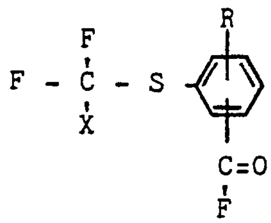

(I)

dans laquelle X représente un atome de chlore ou de fluor, R représente un atome d'hydrogène, un atome d'halogène ou un reste aliphatique, caractérisé en ce que l'on fait réagir des chlorures de trichlorométhylthiobenzoyle de formule

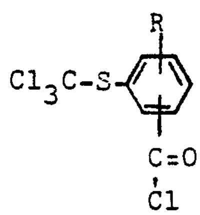

(II)

dans laquelle R a les significations indiquées ci-dessus, avec 1,2 à 100 moles de fluorure d'hydrogène par mole du composé de départ de formule II

a) à une température de 40 à 130 °C et une pression de 2 à 30 bars pour la préparation des fluorures de trifluorométhylthiobenzoyle, ou bien

b) à une température de 10 à 40 °C et une pression de 1 à 10 bars pour la préparation des fluorures de difluoromonochlorométhylthiobenzoyle.

2. Fluorures de fluorométhylthiobenzoyle de formule

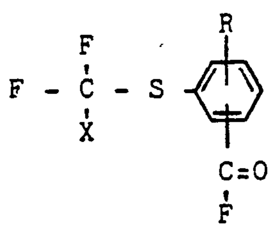

(I)

dans laquelle X représente un atome de chlore ou de fluor, R un atome d'hydrogène, un atome d'halogène ou un reste aliphatique.